# EUROPEAN PATENT SPECIFICATION

(11) **EP 2 771 016 B1**
(45) Date of publication and mention of the grant of the patent: **20.04.2016**
(21) Application number: 12788250.4
(22) Date of filing: 25.10.2012
(51) Int. Cl.: A61K 31/365, A61K 31/7048, A61P 31/04

(54) **PRIMYCIN AND COMPONENTS THEREOF FOR USE IN THE TREATMENT OR PREVENTION OF INFECTIONS CAUSED BY SPECIFIC PATHOGENS**
PRIMYCIN UND BESTANDTEILE DARAUS ZUR VERWENDUNG BEI DER BEHANDLUNG ODER PRÄVENTION VON INFEKTIONEN DURCH SPEZIFISCHE KRANKHEITSERREGER
PRIMYCINE ET SES CONSTITUANTS POUR UNE UTILISATION DESTINÉE AU TRAITEMENT OU À LA PRÉVENTION D'INFECTIONS PROVOQUÉES PAR DES PATHOGÈNES SPÉCIFIQUES

(30) Priority: 25.10.2011 HU P1100597
(43) Date of publication of application: 03.09.2014
(73) Proprietor: Pannonpharma Gyógyszergyártó Zrt., 7720 Pécsvárad (HU)
(72) Inventor: FEISZT, Péter, H-7626 Pécs (HU); EMÖDY, Levente, H-7622 Pécs (HU); PALLOS, József Péter, H-1221 Budapest (HU); JUHÁSZ, Ákos, H-6000 Kecskemét (HU); SEFFER, Dénes, H-7720 Pécsvárad (HU); SEFFERNÉ SZALAI, Mária, H-7720 Pécsvárad (HU); PÉNZES, Ágota, H-9600 Sárvár (HU)
(74) Representative: Fehérvari, Flora
(86) International application number: PCT/HU2012/000111
(87) International publication number: WO 2013/061101

(56) References cited:
- WO-A1-2011/051741
- HU-B- 195 514
- HU-B- 196 309
- US-A- 3 949 077
- US-A- 4 404 189
- US-A- 4 873 348
- VALYI-NAGY T ET AL: "Primycin, a new antibiotic", NATURE: INTERNATIONAL WEEKLY JOURNAL OF SCIENCE, NATURE PUBLISHING GROUP, UNITED KINGDOM, vol. 174, no. 4441, 11 December 1954 (1954-12-11), pages 1105-1106, XP009165902, ISSN: 0028-0836
- INOUE M ET AL: "Enhancement of antibacterial activity of beta-lactam antibiotics by [P2W18O62]<6->, [SiMo12O40]<4->, and [PTi2W10O40]<7-> against methicillin-resistant and vancomycin-resistant Staphylococcus aureus", JOURNAL OF INORGANIC BIOCHEMISTRY, ELSEVIER INC, US, vol. 100, no. 7, 1 July 2006 (2006-07-01), pages 1225-1233, XP027900264, ISSN: 0162-0134 [retrieved on 2006-07-01]
- FERRARA ET AL: "Treatment of hospital-acquired pneumonia caused by methicillin-resistant Staphylococcus aureus", INTERNATIONAL JOURNAL OF ANTIMICROBIAL AGENTS, ELSEVIER SCIENCE, AMSTERDAM, NL, vol. 30, no. 1, 8 June 2007 (2007-06-08), pages 19-24, XP022109120, ISSN: 0924-8579, DOI: 10.1016/J.IJANTIMICAG.2007.02.011
- MASCITTI K B ET AL: "Preferred treatment and prevention strategies for recurrent community-associated methicillin-resistant Staphylococcus aureus skin and soft-tissue infections: A survey of adult and pediatric providers", AMERICAN JOURNAL OF INFECTION CONTROL, C.V. MOSBY CO., ST. LOUIS, MO, US, vol. 38, no. 4, 1 May 2010 (2010-05-01), pages 324-328, XP027043064, ISSN: 0196-6553 [retrieved on 2010-04-24]

## Description

The invention refers to primycin or a primycin component represented by general formula (I) or a combination of primycin components, for use in the treatment or prevention of infections caused by Gram-positive bacteria resistant to methicillin and/or vancomycin and/or mupirocin or by penicillin-resistant streptococci. The invention also covers antibotic compositions containing these active agents.

### Background of the invention

Primycin is a macrolide antibiotic complex comprising more than 20 components, 90% of which consists of nine main components belonging to three major groups.
The first report on primycin was published in 1954 by Vályi-Nagy et al. who isolated the substance from *Streptomyces primycini* cultures [Vályi-Nagy T, Uri J, Szilágyi I. (1954): Nature; 174, 1105.]. Authors report that purified primycin possesses high antibacterial activity against Gram-positive bacteria, namely *Staphylococcus* and *Mycobacterium* species with a minimal inhibitory concentration (MIC) of 0.02 to 0.06 mg/L. According to the paper, primycin is effective against "resistant" strains but the authors do not denominate antimicrobial agents against which these strains are resistant.

In a subsequent study Vályi-Nagy *et al.* report primycin susceptibility of streptomycin--resistant *Mycobacterium* strains [Vályi-Nagy T, Kelentey B. (1960): Arch Int Pharmacodyn Ther 124: 466-481.].

A subsequent publication states that primycin is effective against Gram-negative species with MIC values hundred times higher than those obtained in case of Gram-positives [Horváth I, Kramer M, Bauer PI et. al. (1979): Arch Microbiol. 121(2):135-139.]. Authors state this referring unfoundedly to the above *Nature* article (since the latter does not contain such information).

In the same year, a clear denial of previous statement appeared in a publication on the crystallization of primycin [Uri JV, Actor P. (1979): J Antibiot (Tokyo) Nov; 32(11):1207--1209.]. In this paper both crystalline primycin and its amorphous predecessor are reported to be ineffective against Gram-negative species. This article also confirms primycin susceptibility of isolates of *S*. *aureus* and *Streptococcus faecalis* (i.e. *Enterococcus faecalis* according to the current nomenclature). The MICs reported for these species were in the range of 0.25 to 0.5 µg/ml except for *S*. *aureus* ATCC 25923 strain, which was not inhibited even at 2 µg/ml, i.e. at the highest primycin concentration tested. The authors found no remarkable difference in the efficacy of amorphous and crystalline primycin.

In a subsequent study [Uri JV. (1986): Acta Microbiol Hung; 33(2):141-146] MIC values for crystalline primycin were measured in media possessing different pH values (i.e. 6, 7.3 and 8, respectively) on large collections of clinical isolates of *S*. *aureus, S. epidermidis, E. faecalis* (that time designated as *S. faecalis*) and laboratory strain *Listeria monocytogenes.* All investigated strains were inhibited with MIC values of 0.12 to 0.5 µg/ml, which were only slightly influenced by the actual pH. For laboratory strain *L. monocytogenes* the MIC value of 0.25 µg/ml was measured. The author also stated that the MIC values of primycin were independent of the status of resistance to other antibiotics, however, he did not denominate the latter.

In another publication on the chemical structure of primycin [Frank J, Dékány Gy, Pelczer I, ApSimon W J (1987): Tetrahedron Letters; 28:2759-2762] authors describe primycin as a multicomponent mixture and present the chemical structures of nine main components. They classify primycin in the macrolide antibiotic group.

A review [Nógrádi M. (1988): Drugs of Today 24(8): 563-566] repeats earlier statements according to which primycin is highly effective against Gram-positive bacteria, including "resistant and polyresistant" strains, and in high concentrations, also against Gram--negative bacteria. MIC ranges of primycin for specific genera were summarized as follows: 0.02 to 0.1 mg/L for *Staphylococcus spp., Streptococcus spp., Bacillus spp., Mycobacterium spp., Listeria spp., Sarcina spp., Sporosarcina spp., Propionibacterium spp.,* 1 to 10 mg/L for *Neisseria spp., Enterococcus spp. Vibrio spp.,* 10 to 25 mg/l for *Shigella spp.* and 25 to 50 mg/l for *Pasteurella spp.* and *Serratia spp..* MIC values of primycin for specific bacterial strains were compared with those of ampicillin, erythromycin, oxytetracycline, streptomycin and clindamycin, as shown in table 1.

**Table 1: Efficacy of primycin and reference antibiotics against different bacteria bv Nógrádi**

| **Organism** | **MIC (mg/l)** | | | | | |
|---|---|---|---|---|---|---|
| | **Primycin** | **Ampicillin** | **Erythromycin** | **Oxytetracycline** | **Streptomycin** | **Clindamycin** |
| *Staphylococcus epidermidis* **7586** | 0.78 | <0.5 | <0.5 | 62.5 | 2.0 | 31.3 |
| *Staphylococcus aureus* 7503 | 0.39 | <0.5 | >250 | 1.0 | 2.0 | >250 |
| *Diphteroides* 8264 | 50.0 | 31.3 | >250 | 31.3 | >250 | 250 |
| *Staphylococcus saprophyticus* 8454 | 0.39 | <0.5 | 1.0 | <0.5 | 1.0 | 15.6 |
| Serotype β *Streptococcus* 8252 | 1.56 | <0.5 | 0.5 | 1.0 | 3.9 | 125 |
| *Streptococcus mitis* 5728 | 50.0 | 7.8 | 250 | 1.0 | 250 | >250 |
| *Staphylococcus hominis* 7835 | 0.39 | <0.5 | 250 | 31.3 | 2.0 | 31.3 |
| *Streptococcus simulans* 7866 | 0.39 | <0.5 | 7.8 | 2.0 | 2.0 | 31.3 |
| *Micrococcus spp.* | 0.78 | <0.5 | <0.5 | 7.8 | 3.9 | 15.6 |
| *Staphylococcus capitis* | 0.78 | <0.5 | <0.5 | 31.3 | 3.9 | 31.3 |
| *Staphylococcus aureus* ATCC 25923 | 25.0 | <0.5 | 1.0 | <0.5 | 3.9 | 62.5 |
| *Escherichia coli* B 1218 | 50.0 | 2.0 | 62.5 | 1.0 | 15.6 | >250 |
| *Enterobacter cloacae* B 520 | 50.0 | 7.8 | 250 | 2.0 | 7.8 | >250 |
| *Bacillus subtilis* ATCC 6633 | 0.39 | <0.5 | <0.5 | <0.5 | 1.0 | 15.6 |
| *Propionibacterium acnes* | <0.1 | 1.0 | <0.5 | <0.5 | <0.5 | 0.5 |

This review cited another study [Nógrádi M. (1988): Drugs of Today 24(8): 563-566] according to which 1% of a collection of 279 erythromycin-sensitive *Staphylococcus spp.* strains was primycin-resistant (MIC≥5 mg/L), while 13% of 71 erythromycin-resistant staphylococci presented primycin resistance.

In a handbook [Bryskier A. (2005) ASM Press Washington DC: Antimicrobials - antibacterials and antifungals, chapter titled "Primycin", pages 1179-1180] primycin is characterized as being effective against *S. aureus* and coagulase-negative staphylococci but showing only moderate efficacy against enterococci and *S. pyogenes.* The author mentions that primycin is effective against *Bacillus spp.* and *Micrococcus spp.,* but ineffective against *Corynebacterium spp., Enterobacteriaceae* species, *Pseudomonas aeruginosa,* yeasts and dermatophytes. The author presents a table which partly overlaps with the one included in the cited Nógrádi article, see table 2.

**Table 2: Efficacy of primycin and reference antibiotics against different bacteria by Bryskier**

| Organism(s) | MIC (mg/l) | | | | |
|---|---|---|---|---|---|
| | Primycin | Ampicillin | Erythromycin A | Tetracycline | Clindamycin |
| *S. aureus* | 0.39 | <0.5 | >250 | 1.0 | >250 |
| *S. aureus* ATCC 25923 | 25 | <0.5 | 1.0 | <0.5 | 62.5 |
| *Staphylococcus epidermidis* | 0.78 | <0.5 | <0.5 | 62.5 | 31.3 |
| *S. pyogenes* | 1.56 | <0.5 | 0.5 | 1.0 | 125 |
| *Streptococcus mitis* | 50 | 7.8 | 250 | 1.0 | >250 |
| *Micrococcus* | 0.78 | <0.5 | <0.5 | 7.8 | 15.6 |
| Coryneform(s) | 50 | 31.3 | >250 | 31.3 | 250 |
| *B. subtilis* ATCC 6633 | 0.39 | <0.5 | <0.5 | <0.5 | 15.6 |
| *Escherichia coli* | 50 | 2.0 | 62.5 | 1.0 | >250 |
| *Enterobacter cloacae* | 50 | 7.8 | 250 | 2.0 | >250 |
| *Propionibacterium acnes* | <0.1 | 1.0 | <0.5 | <0.5 | 0.5 |

Hungarian Patent No. 153593 (Owner: Chinoin Gy6gyszer és Vegyészeti Termékek Gyára Rt., Budapest) describes an improved process for the preparation of primycin. This patent concerns an industrial-scale process utilizing a *Thermopolyspora galeriensis* strain, by which primycin can be produced more effectively. Antibiotic-producing properties of the new industrial strain surpass those of the *Streptomyces primycini* strains used previously for this purpose.

Hungarian Patents Nos. 195514, 196309 and 196822 (Owner for each: Chinoin Gy6gyszer és Vegyészeti Termékek Gyára RT, Budapest) disclose information on microbial pathogens against which primycin is applicable. In each document it is declared in general that primycin is effective primarily against Gram-positive pathogens.

Hungarian Patent No. 196309 describes synergistic effect of dual or triple combinations of primycin components A1, B1 and C 1 in various mass ratios. The antimicrobial effect of the individual primycin components is also described, which proves their independent applicability.

US Patent No. US4873348 discloses nine components of primycin and describe the outstanding efficacy of oxypricin (primycin component C1).

Summary of Product Characteristics of Ebrimycin gel, a human medicinal product containing primycin as active substance, includes the following statement: Based on literary data, synergistic interaction is present in dual or multiple combinations of primycin with agents selected from the group of oxytetracycline, streptomycin and oxacillin, or from penicillin and vancomycin. Antagonistic interaction of primycin is reported with novobiocin, erythromycin, chloramphenicol, fuzidine.

Synergistic interactions of primycin with other antibiotics are described in the following documents as well.

According to US Patent No. US3949077 primycin shows synergistic effect in dual combination with viomycin, streptomycin, oxacillin, neomycin or oxytetracycline or in triple combination with neomycin and oxytetracycline.

US Patent No. US4404189 discloses synergistic combinations of primycin with sisomicin and/or doxycyclin.

### The problem to be solved by the invention

Antibiotic resistance is an emerging problem in the therapy of bacterial infections. Resistance against widely used antibiotics is already so frequent that new therapy protocols have to be elaborated for patients affected.

The scientific literare on the applicability of primycin is rather incomplete, still it suggests that primycin is efficient primarily against Gram-positive pathogens, particularly against *Staphylococcus* species.

According to the above cited Hungarian Patents Nos. 195514, 196309 and 196822 primycin is also active against polyresistant strains. However the credibility of this statement cannot be verified; the antibiotics to which these bacterial strains show resistancy were not specified, moreover some of the strains titled in these patents as being polyresistant are no longer available (i.e. no information is available concerning their "polyresistance"). Even a strain that was not polyresistant at the filing date of these patents was mentioned, as seen in table 3. It can be stated however, that these strains cannot be characterized by the antibiotic resistances discussed in the present description.

Furthermore, the presently studied Gram-positive bacterial strain *Streptococcus pneumoniae* was not mentioned in the above patents.

**Table 3: Gram-positive bacterial strains titled as polyresistant in the patent literature on the efficacy of primycin**

| **Bacterial strains titled as polyresistant in earlier patents** | | **Note** |
|---|---|---|
| **Species** | **Strain id. number** | |
| *Bacillus subtilis* | ATCC 6633 | Not polyresistant. A strain often used to estimate the efficacy of antibiotics. |
| *Bacillus subtilis* | CCM 1718, ATCC 9799 | Not resistant against methicillin, mupirocin or vancomycin. Polyresistance is not confirmed. |
| *Bacillus cereus* | CCM 2010, ATCC 14579 | |
| *Bacillus licheniformis* | CCM 2182 | |
| *Bacillus licheniformis* | CCM 2205, ATCC 9789 | |
| *Listeria monocytogenes* | CCM 5576, ATCC 19111 | |
| *Micrococcus luteus* | DSM 20030, ATCC 4698 | |
| *Sporosarcina ureae* | DSM 317 | |
| *Staphylococcus aureus* | CCM 885, ATCC 12600 | |
| *Staphylococcus aureus* | CCM 2317 | |
| *Staphylococcus aureus* | CCM 2514 | Not available. |
| *Staphylococcus aureus* | CCM 2326 | Methicillin-, mupirocin- or vancomycin-resistance is not present. Polyresistance is not confirmed. |
| *Staphylococcus aureus* | CCM 2515 | |
| *Staphylococcus epidermidis* | CCM 2271 | Not available. |
| *Staphylococcus aureus* | Smith ATCC 12715, 19636 | Methicillin-, mupirocin- or vancomycin-resistance is not present. Polyresistance is not confirmed. |
| *Staphylococcus aureus* | DSM 20231, ATCC 12600 | |
| *Streptococcus, faecalis* | CCM 1875, ATCC 11700 | |
| *Streptococcus agalactiae* | CCM 5534 | |
| *Streptococcus agalactiae* | CCM 5153 | |
| *Streptococcus disgalactiae* | CCM 5548, ATCC 9026 | |
| *Streptococcus disgalactiae* | ATCC 9926 | |
| *Micrococcus flavus* | ATCC 10240 | |

The purpose of our work was to reveal yet unknown application opportunities of primycin, primarily against well-characterized pathogen groups with specified antibiotic resistance causing significant difficulties in the therapy. The primycin susceptibility of these pathogen groups and thus, the applicability of primycin against them have not been described until now.

Presently, the following strains cause the most and major problems in the therapy: methicillin-resistant *Staphylococcus aureus* (MRSA) and coagulase-negative staphylococci (MRCoNS), vancomycin-resistant enterococci (VRE), vancomycin-intermediate and vancomycin-resistant *Staphylococcus aureus* (VISA and VRSA), the latter strains possess methicillin-resistance as well. To eradicate asymptomytic MRSA/VISA/VRSA-colonization of the hospital staff, mupirocin is usually used, which resulted in selection of mupirocin--resistant variations of these pathogens. The afore-mentioned resistances are often accompanied by cross-resistances to several agents, including macrolide antibiotics and thus theoretically to primycin as well. This theory is supported by the fact that, while earlier publications did not mention primycin-resistance, the above cited paper by Nógrádi (1988) reported that 1% of a collection of 279 erythromycin-sensitive *Staphylococcus spp.* strains was primycin-resistant (MIC≥5 mg/L), while 13% of 71 erythromycin-resistant staphylococci presented primycin resistance, which indicates a connection between erythromycin- and primycin-resistance.

In addition, primycin resistance appeared in case of *Staphylococcus aureus* ATCC 25923 strain, according to earlier publications. In view of the fact that primycin has been on the market since the issuance of the papers that already mentioned primycin-resistant strains, there has been a chance for selection and spreading of primycin-resistant strains.

Besides those mentioned above, currently upcoming *Streptococcus pneumoniae* strains with decreased penicillin susceptibility or penicillin-resistance are also of great clinical importance.

In respect of the adequate therapy and the related clinical outcome, patients with infections caused by bacteria possessing the above-described resistance(s) constitute patient groups clearly separated from those infected by susceptible strains of the same species.

By testing the primycin-susceptibility of bacteria possessing the above-described resistance(s) it can be decided whether these strains show cross-resistance to primycin. If not, primycin can afford new opportunity in therapy and prevention of infections caused by these bacteria.

### General description of the invention

The invention relates to primycin or a primycin component represented by general formula (I) or a combination of primycin components for use in the treatment or prevention of infections caused by methicillin-resistant *Staphylococcus aureus* (MRSA), methicillin-resistant coagulase-negative *Staphylococcus spp.* (MRCoNS), vancomycin-intermediate or vancomycin-resistant *Staphylococcus aureus* (VISA, VRSA), mupirocin-resistant *Staphylococcus spp.,* vancomycin-resistant *Enterococcus spp.* (VRE) or penicillin-resistant *Streptococcus pneumoniae* strains.

The invention covers pharmaceutical compositions containing primycin or a primycin component represented by general formula (I) or a combination thereof for use in the treatment or prevention of infections caused by the bacterial strains defined above.

The invention also covers antibiotic compositions containing primycin or a primycin component represented by general formula (I) or a combination thereof for use in the treatment or prevention of infections caused by the bacterial strains defined above.

### Detailed description of the invention

Primycin components for use according to the invention are represented by general formula (I): 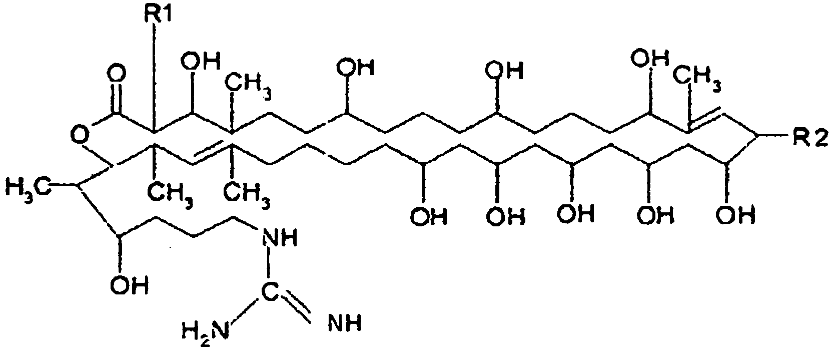 wherein R1 is n-butyl, n-pentyl or n-hexyl and R2 is arabinosyl, H or OH.

Said primycin components are in basic form or in salt form. Among them preferred are sulphate salts, but they can form salts with other inorganic or organic acids, such as acetate.

Denominations and substituents R1 and R2 of the primycin components of formula (I) are as follows:

| Component | R1 | R2 |
|---|---|---|
| A1 (chinopricin) | n-butyl | -arabinosyl |
| A2 (midopricin) | n-pentyl | -arabinosyl |
| A3 (metipricin) | n-hexyl | -arabinosyl |
| B1 (hydropricin) | n-butyl | -H |
| B2 (hymipricin) | n-pentyl | -H |
| B3 (hymetipricin) | n-hexyl | -H |
| C1 (oxypricin) | n-butyl | -OH |
| C2 (oxymipricin) | n-pentyl | -OH |
| C3 (oxymetipricin) | n-hexyl | -OH |

The chemical name of primycin component A1 is for example 18-arabinosyl-2-butyl--3,7,11,15,19,21,23,25,27,37-decahydroxy-4,16,32,34,36-pentamethyl-tetraconta-16,32--diene-35-O-lacton-40-guanidin-sulphate or {5-/19-(α-D-Arabinofuranosyloxy)-35-butyl--10,12,14,16,18,22,26,30,34-nonahydroxy-3,5,21,33-tetramethyl-36--oxooxacyclohexatriaconta-4,40-diene-2-il/-4-hydroxyhexyl}-guanidin-sulphate.

The term "combination of primycin components" used in the present description refers to a mixture of at least two primycin components in any ratio.

In one embodiment, the invention relates to combination of primycin components A1 and B1 in a ratio of 1:3 to 3:1, for example 1:3, 1:1 or 3:1 (molar ratios).

In another embodiment, the invention relates to combination of primycin components A1 and C1 in a ratio of 1:3 to 3:1, for example 1:3, 1:1 or 3:1.

In another embodiment, the invention relates to combination of primycin components C1 and B1 in a ratio of 1:3 to 3:1, for example 1:3, 1:1 or 3:1.

In another embodiment, the invention relates to combination of primycin components A1, B1 and C1 in a ratio of 4:3:3 to 7:2:1, for example 4:3:3, 5:2.5, 6:2:2 or 7:2:1.

Primycin for use according to the present invention can be manufactured via the process described in the above Hungarian Patent No. 153593 (Chinoin). The primycin components can be prepared by the processes described in Hungarian Patents Nos. 195514 (published as T/39186) and 196425 (published as T/39187).

The pharmaceutical composition according to the invention contains as active agent primycin or a primycin component represented by general formula (I) or a composition of primycin components together with at least one pharmaceutical acceptable carrier or additive.

The term "pharmaceutical composition" used in the present description refers to any composition containing together with carriers and/or additives, an active agent useable for retaining or recovering health of a human or an animal, independently of the way of administration, including dietary supplements, functioned food nutraceutical food and the like.

The pharmaceutical compositions according to the invention can be in any commonly used forms, for example solid, semisolid or liquid forms and contain commonly used excipients and/or vehicle materials determined by the given form.

Solid pharmaceutical forms can be for example tablets, capsules or coated tablets, semisolid forms can be ointments, creams or gels, liquid forms can be solutions, suspensions or emulsions.

Solid forms, like tablets capsules or coated tablets can be administered orally. Further oral preparations are liquid compositions like solutions, suspensions or emulsions. Powder mixtures added to forage or solutions added to drinking water can be used for veterinary applications.

Parenterally applicable pharmaceutical forms are aqueous solutions, suspensions or emulsions.

Topically or locally applicable forms are powders, ointments, gels or aqueous solutions, suspensions or emulsions. Semisolid and liquid forms are preferably used on the surfaces of mucous membranes.

The pharmaceutical compositions are prepared by mixing the active substance with non--toxic, inert vehicles and/or excipients commonly used in pharmaceutics.

Conventionally used vehicle materials are for example water, gelatine, lactose, starch, magnesium-stearate, stearic acid, glycols, alcohols, vegetable oils, etc. In case of creams and ointments vaseline, liquid paraffin, lanoline, polyethylene-glycols, alcohols and any mixtures thereof can be used as vehicles. Excipients conventionally used in pharmaceutics are for example preservatives, buffers, moisturizers, emulsifiers, colorants, flavorings, etc.

The pharmaceutical compositions exemplified above and preparations thereof are well known e.g. from the Remington's Pharmaceutical Sciences manual [18. issue, Mack Publishing Co., Easton, USA (1990)].

The pharmaceutical compositions according to the invention are preferably applied on the surfaces of skin or mucous membrane. Advantageously, they are in the forms of creams, ointments, gels, solutions or aqueous suspensions, the latter are preferably in the forms of eye drops, nasal drops or nasal spray, lotion, and can contain commonly used vehicles and/or excipients adequate to the given form.

The pharmaceutical compositions according to the invention contain primycin or a primycin component represented by general formula (I) or a combination of primycin components in an amount adequate to the specific form, the way of administration, and the MIC value of the target microorganism.

The topically applicable pharmaceutical compositions according to the invention contain primycin or a primycin component represented by general formula (I) or a combination of primycin components preferably in a concentration range of 0.01 to 10 mg/g, more preferably of 0.1 to 1.0 mg/g.

An aqueous suspension applicable on mucous membranes contains preferably 50 to 150 mg/g of polyvinyl alcohol, 0.2 to 1.2 mg/g of anhydrous NaH₂PO₄, 4.0 to 5.0 mg/g of Na₂HPO₄.2H₂O, 6 to 7 mg/g of polysorbate, 1.0 to 1.5 mg/g of disodium edetate, 5.0 to 6.0 mg/g of sodium chloride and water for injection.

An alcoholic gel according to the invention preferably contains 7.0 to 14 mg/g of carbomera, 15 to 20 mg/g of triethanolamine, 15 to 20 mg/g of polysorbate, 50 to 70 mg/g of isoadipate, 400 to 600 mg/g ethanol (96%) and purified water.

Compositions applicable on the surfaces of mucous membrane can be used for example to eradicate asymptomatic methicillin- and/or vancomycin- and/or mupirocin-resistant nasal colonisations of the hospital personnel. In this manner, the personnel cease to be an infection source.

The term "antibacterial composition" used in the present description refers to any composition containing primycin or a primycin component represented by general formula (I) or a combination of primycin components for use according to the present invention, together with carriers and/or vehicles commonly used in sanitary and hygienic products. The forms and concentration ranges of these compositions are as described for pharmaceutical compositions above.

### Bacteriological studies

The research work during which the present invention was elaborated, was carried out in the Department of Medical Microbiology and Immunology, Medical School, University of Pécs.

Susceptibilities of a total of 105 clinical isolates of genera *Staphylococcus, Streptococcus* and *Enterococcus* and 11 international reference strains were tested to primycin, vancomycin, gentamicin, erythromycin, tobramycin, neomycin, ofloxacin, and oxytetracyclin. Identification and characterization of resistance patterns of these bacteria were performed by standard methods.

When forming the test groups we attached great importance to the increasing incidence of drug-resistant microbes causing therapeutic problems. Most important of them are methicillin-resistant *Staphylococcus aureus* (MRSA), methicillin-resistant coagulase-negative *Staphylococcus spp.* (MRCoNS), penicillin-resistant *Streptococcus pneumoniae* (pneumococcus), vancomycin-resistant *Enterococcus spp.* (VRE). Pneumococcus isolates represented various serotypes, VRE isolates belonged to various species. Moreover, vancomycin-resistance of VRE isolates was based on different genetic backgrounds. Antibiotic-resistant strains were also represented among international reference strains, for example methicillin-resistant *Staphylococcus aureus* ATCC 43300 (MRSA), *Staphylococcus aureus* with decreased vancomycin susceptibility ATCC 700698 and 700699 (vancomycin intermediate *Staphylococcus aureus -* VISA), mupirocin-resistant *Staphylococcus aureus* ATCC BAA1708 and vancomycin-resistant *Enterococcus faecalis* ATCC 51299 (VRE).

Susceptibility tests were performed according to the standard microdilution method (NCCLS (CLSI) M7-A7).

Efficacy of antibiotics was tested at concentrations presented in Table 4.

**Table 4: Antibiotic concentrations tested (sensitive MIC range, intermediate range, resistant range).**

| **Test agents** | **Final concentrations tested (mg/L)** | | | | | | | | | |
|---|---|---|---|---|---|---|---|---|---|---|
| Primycin | **8** | **4** | **2** | **1** | **0.5** | **0.25** | **0.12** | **0.06** | **0.03** | **0.015** |
| Ofloxacin | 16 | 8 | *4* | **2** | **1** | **0.5** | **0.25** | **0.12** | **0.06** | **0.03** |
| Tobramycin | 32 | 16 | 8 | **4** | **2** | **1** | **0.5** | **0.25** | **0.12** | **0,06** |
| Oxytetracycline | 32 | 16 | *8* | **4** | **2** | **1** | **0.5** | **0.25** | **0.12** | **0.06** |
| Erythromycin | 16 | 8 | 4 | *2* | *1* | **0.5** | **0.25** | **0,12** | **0.06** | **0.03** |
| Gentamicin | 16 | 8 | **4** | **2** | **1** | **0.5** | **0.25** | **0.12** | **0.06** | **0.03** |
| Neomycin | 32 | 16 | **8** | **4** | **2** | **1** | **0.5** | **0.25** | **0.12** | **0.06** |
| Vancomycin | 32 | 16 | *8* | **4** | **2** | **1** | **0.5** | **0.25** | **0.12** | **0.06** |

The lowest concentration at which no bacterial growth was observed after the incubation period is the Minimal Inhibitory Concentration (MIC). The MIC value is inversely proportional to the antimicrobial efficacy. MIC values of antimicrobial agents can differ by species or even by bacterial strains within species. MIC₅₀ and MIC₉₀ values (the lowest concentrations inhibiting 50 or 90%, respectively, of the isolates) were calculated from a minimum of 10 strains of a microbe group formed according to objective aspects.

The concentration ranges of reference antimicrobial agents tested parallel with primycin were selected to involve the MIC interpretive breakpoints reflecting the clinical applicability (Table 4).

Susceptibilities of bacterial strains and groups thereof to vancomycin, gentamicin, erythromycin, tobramycin, neomycin, ofloxacin and oxytetracycline were in good correlation with literary data (Table 5). Among these agents only vancomycin showed high and extended efficacy.

Resistance patterns of MRSA, MRCoNS and VRE isolates show decreased susceptibility to the reference antibiotics, including erythromycin, which belongs to the macrolide antibiotic group. As it was expected, methicillin- and vancomycin-resistance often coexistes with resistance to the other antibiotics.

We have surprisingly found that primycin inhibited all the tested bacteria with MIC₉₀ values of 0.06 to 1 mg/L depending on species, including MRSA, MRCoNS and VRE isolates. Surprisingly, no cross-resistance to primycin appeared with methicillin-resistance or with vancomycin-resistance, despite the fact that primycin belongs to the macrolide group. Cross-resistance to primycin was not experienced among erythromycin-resistant isolates either, as it can be seen in table 5.

**Table 5: Efficacy of primycin and comparative agents against clinical isolates of Gram-positive bacteria**

| **Antimicrobials** | **Bacteria (number of isolates)** | **MIC (mg/L)** | | | **Ratio of Sensitive/Total** |
|---|---|---|---|---|---|
| | | **Range** | **50**% | **90%** | |
| Primycin | *Staphylococcus aureus*, methicillin susceptible (10) | 0.06 - 0.06 | 0.06 | 0.06 | 10/10 |
| | *Staphylococcus aureus,* methicillin resistant (10) | 0.06 - 0.06 | 0.06 | 0.06 | 10/10 |
| | Coagulase-negative *Staphylococcus sp.,* methicillin susceptible (10) | 0.03 - 0.06 | 0.03 | 0.06 | 10/10 |
| | Coagulase-negative *Staphylococus sp.,* methicillin resistant (10) | 0.03 - 0.06 | 0.06 | 0.06 | 10/10 |
| | *Enterococcus sp.,* vancomycin susceptible (20) | 0.5 - 1 | 0.5 | 0.5 | 20/20 |
| | *Enterococcus sp.,* decreased vancomycin susceptibility (5) | 0.25 - 0.5 | - | - | 5/5 |
| | *Streptococcus pneumoniae,* penicillin susceptible (10) | 0.25 -1 | 0.5 | 0.5 | 10/10 |
| | *Streptococcus pneumoniae,* penicillin resistant (10) | 0.5 - 1 | 0.5 | 1 | 10/10 |
| | *Streptococcus viridans sp. (20)* | 0.5 - 1 | 1 | 1 | 20/20 |
| Vancomycin | *Staphylococcus aureus,* methicillin susceptible (10) | 0.5 - 2 | 1 | 1 | 10/10 |
| | *Staphylococcus aureus,* methicillin resistant (10) | 0.5 - 1 | 1 | 1 | 10/10 |
| | Coagulase-negative *Staphylococus sp.,* methicillin susceptible (10) | 1 - 2 | 1 | 2 | 10/10 |
| | Coagulase-negative *Staphylococus sp.,* methicillin resistant (10) | 1 - 2 | 2 | 2 | 10/10 |
| | *Enterococcus sp.,* vancomycin susceptible (20) | 1 - 4 | 1 | 2 | 20/20 |
| | *Enterococcus sp.,* decreased vancomycin susceptibility (5) | 8 - >32 | - | - | 0/5 |
| | *Streptococcus pneumoniae,* penicillin susceptible (10) | 0.25 - 0.5 | 0.5 | 0.5 | 10/10 |
| | *Streptococcus pneumoniae,* penicillin resistant (10) | 0.25 - 0.5 | 0.5 | 0.5 | 10/10 |
| | *Streptococcus viridans sp. (20)* | 0.5 - 1 | 0.5 | 1 | 10/20 |
| Gentamicin | *Staphylococcus aureus,* methicillin susceptible (10) | 0.25 - 0,5 | 0.5 | 0.5 | 10/10 |
| | *Staphylococcus aureus,* methicillin resistant (10) | 0.25 - 1 | 0.5 | 0.5 | 10/10 |
| | Coagulase-negative *Staphylococus sp.,* methicillin susceptible (10) | 0.03 - 8 | 0.125 | 4 | 9/10 |
| | Coagulase-negative *Staphylococcus sp.,* methicillin resistant (10) | 0.06 - >16 | 0.125 | >16 | 6/10 |
| | *Enterococcus sp.,* vancomycin susceptible (20) | 4 - >16 | 8 | >16 | 1/20 |
| | *Enterococcus sp.,* decreased vancomycin susceptibility (5) | 8 - 16 | - | - | 0/5 |
| | *Streptococcus pneumoniae,* penicillin susceptible (10) | 8 - 16 | 16 | 16 | 0/10 |
| | *Streptococcus pneumoniae,* penicillin resistant (10) | 8 - >16 | 16 | >16 | 0/10 |
| | *Streptococcus viridans sp. (20)* | 2 - >16 | 8 | 16 | 9/20 |
| Erythromycin | *Staphylococcus aureus,* methicillin susceptible (10) | 0.125 - >16 | 0.125 | >16 | 7/10 |
| | *Staphylococcus aureus,* methicillin resistant (10) | 0.25 - >16 | >16 | >16 | 1/10 |
| | Coagulase-negative *Staphylococus sp.,* methicillin susceptible (10) | 0.03 - >16 | 0.125 | >16 | 8/10 |
| | Coagulase-negative *Staphylococus sp.,* methicillin resistant (10) | 0.125 - >16 | 0.25 | >16 | 5/10 |
| | *Enterococcus sp.,* vancomycin susceptible (20) | 0.03 - >16 | 1 | >16 | 13/20 |
| | *Enterococcus sp.,* decreased vancomycin susceptibility (5) | 0.06 - >16 | - | - | 2/5 |
| | *Streptococcus pneumoniae,* penicillin susceptible (10) | 0.03 - >16 | 0.03 | 0.03 | 9/10 |
| | *Streptococcus pneumoniae,* penicillin resistant (10) | 0.03 - >16 | 2 | >16 | 2/10 |
| | *Streptococcus viridans sp. (20)* | 0.03 - >16 | 1 | >16 | 10/20 |
| Oxytetracyclin | *Staphylococcus aureus*, methicillin susceptible (10) | 0.25 - 32 | 0.25 | 32 | 8/10 |
| | *Staphylococcus aureus*, methicillin resistant (10) | 0.125 - 0.25 | 0.25 | 0.25 | 10/10 |
| | Coagulase-negative *Staphylococus sp.,* methicillin susceptible (10) | 0.125 - >32 | 0.25 | 1 | 9/10 |
| | Coagulase-negative *Staphylococus sp.,* methicillin resistant (10) | 0.125 - >32 | 1 | >32 | 8/10 |
| | *Enterococcus sp.,* vancomycin susceptible (20) | 0.25 - >32 | 16 | 32 | 6/20 |
| | *Enterococcus sp.,* decreased vancomycin susceptibility (5) | 0.25 - 16 | - | - | 2/5 |
| | *Streptococcus pneumoniae,* penicillin susceptible (10) | 1 - 8 | 1 | 8 | 8/10 |
| | *Streptococcus pneumoniae,* penicillin resistant (10) | 4 - >32 | 32 | >32 | 1/10 |
| | *Streptococcus viridans sp. (20)* | 1 - >32 | 4 | >32 | 10/20 |
| Tobramycin | *Staphylococcus aureus,* methicillin susceptible (10) | 0.5 - 1 | 0.5 | 0.5 | 10/10 |
| | *Staphylococcus aureus,* methicillin resistant (10) | 0.5 - >32 | 1 | >32 | 6/10 |
| | Coagulase-negative *Staphylococus sp.,* methicillin susceptible (10) | 0.06 - 4 | 0.06 | 4 | 10/10 |
| | Coagulase-negative *Staphylococus sp.,* methicillin resistant (10) | 0.06 - >32 | 0.06 | >32 | 5/10 |
| | *Enterococcus sp.,* vancomycin susceptible (20) | 8 - >32 | 16 | >32 | 0/20 |
| | *Enterococcus sp.,* decreased vancomycin susceptibility (5) | 8 - >32 | - | - | 0/5 |
| | *Streptococcus pneumoniae,* penicillin susceptible (10) | 16 - 32 | 16 | 32 | 0/10 |
| | *Streptococcus pneumoniae,* penicillin resistant (10) | 16 - >32 | 32 | >32 | 0/10 |
| | *Streptococcus viridans sp. (20)* | 4 - 32 | 16 | 32 | 1/20 |
| Neomycin | *Staphylococcus aureus,* methicillin, susceptible (10) | 0.5 - 2 | 1 | 2 | 10/10 |
| | *Staphylococcus aureus,* methicillin resistant (10) | 0.5 - >32 | 1 | >32 | 5/10 |
| | Coagulase-negative *Staphylococus sp.,* methicillin susceptible (10) | 0.06 - 4 | 0.25 | 1 | 10/10 |
| | Coagulase-negative *Staphylococus sp.,* methicillin resistant (10) | 0.06 - >32 | 0.25 | 16 | 8/10 |
| | *Enterococcus sp.,* vancomycin susceptible (20) | 16 - >32 | >32 | >32 | 0/20 |
| | *Enterococcus sp.,* decreased vancomycin susceptibility (5) | 16 - >32 | - | - | 0/5 |
| | *Streptococcus pneumoniae,* penicillin susceptible (10) | 32 - >32 | >32 | >32 | 0/10 |
| | *Streptococcus pneumoniae,* penicillin resistant (10) | >32 - >32 | >32 | >32 | 0/10 |
| | *Streptococcus viridans sp. (20)* | 16 - >32 | >32 | >32 | 0/20 |
| Ofloxacin | *Staphylococcus aureus*, methicillin susceptible (10) | 0.5 - 1 | 0.5 | 1 | 10/10 |
| | *Staphylococcus aureus,* methicillin resistant (10) | 1 - >16 | 16 | >16 | 1/10 |
| | Coagulase-negative *Staphylococus sp.,* methicillin susceptible (10) | 0.25 - 1 | 1 | 1 | 10/10 |
| | Coagulase-negative *Staphylococus sp.,* methicillin resistant (10) | 4 - >16 | >16 | >16 | 0/10 |
| | *Enterococcus sp.,* vancomycin susceptible (20) | 2 - >16 | 4 | >16 | 3/20 |
| | *Enterococcus sp.,* decreased vancomycin susceptibility (5) | 2 - 8 | - | - | 2/5 |
| | *Streptococcus pneumoniae,* penicillin susceptible (10) | 1 - 4 | 2 | 2 | 9/10 |
| | *Streptococcus pneumoniae,* penicillin resistant (10) | 1 - 2 | 1 | 2 | 10/10 |
| | *Streptococcus viridans sp. (20)* | 1 - 8 | 2 | 4 | 14/20 |

Neither the serotype nor the degree of the penicillin susceptibility of *S. pneumoniae* strains influenced the excellent efficacy of primycin, as seen in table 6.

**Table 6: Pimycin susceptibilities of 20 S. pneumoniae isolates of various serotypes and penicillin susceptibility**

| **Penicillin susceptible *S. pneumoniae*** | | | **Penicillin resistant *S. pneumoniae*** | | |
|---|---|---|---|---|---|
| **Serotype** | **MIC (mg/L)** | | **Serotype** | **MIC (mg/L)** | |
| | **Penicillin** | **Primycin** | | **Penicillin** | **Primycin** |
| 19F | 0.03 | 0.5 | 19A | 16 | 0.5 |
| 9(V) | 0.03 | 0.5 | 19A | 8 | 1 |
| 23(F) | 0.015 | 0.25 | 19A | 4 | 0.5 |
| 4 | 0.015 | 0.5 | 19A | 4 | 1 |
| 8 | 0.015 | 0.5 | 19A | 4 | 0.5 |
| 3 | 0.015 | 0.5 | 14 | 4 | 0.5 |
| 7F | 0.015 | 0.5 | 14 | 2 | 0.5 |
| 11(A) | 0.06 | 0.5 | 14 | 2 | 0.5 |
| 6(A) | 0.06 | 1 | 14 | 2 | 0.5 |
| 6(A) | 0.015 | 0.5 | 23(F) | 2 | 0.5 |

Neither the species, nor the degree of the vancomycin-resistance or the type of the resistance gene of vancomycin-resistant enterococci affected the excellent efficacy of primycin, as seen in table 7.

**Table 7: Primycin susceptibilities of Enterococcus isolates with various van resistance genes**

| **Species** | **Resistance gene** | **MIC (mg/L)** | |
|---|---|---|---|
| | | **Vancomycin** | **Primycin** |
| *E. faecalis* | *van* A | >32 | 0.5 |
| *E. faecalis* | *van* C1 | 8 | 0.5 |
| *E. faecium* | *van* B | >32 | 0.25 |
| *E. casseliflavus* | *van* C1 | 16 | 0.5 |
| *E. casseliflavus* | *van* C2 | 8 | 0.5 |

The primycin susceptibilities of ATCC reference strains showed good accord with the susceptibilities of the clinical isolates.

Reference strains *S. aureus* ATCC 43300 (MRSA), *S. aureus* ATCC 700698, and 700699 (VISA), *S. aureus* ATCC BAA1708 (mupirocin-resistant), *E. faecalis* ATCC 51299 (VRE) also proved to be sensitive to primycin to the same extent as it was observed with clinical isolates, as it can be seen in table 8.

**Table 8: Antibacterial activity of primycin against ATCC reference strains**

| **Species** | **Strain** | **Resistance** | **Primycin MIC (mg/l)** |
|---|---|---|---|
| *Staphylococcus aureus* | ATCC 29213 | | 0.06 |
| *Staphylococcus aureus* | ATCC 25923 | | 0.06 |
| *Staphylococcus aureus* | ATCC 43300 | MRSA | 0.06 |
| *Staphylococcus aureus* | ATCC 700698 | VISA | 0.06 |
| *Staphylococcus aureus* | ATCC 700699 | VISA | 0.125 |
| *Staphylococcus aureus* | ATCC BAA 1708 | mupirocin-resistant | 0.06 |
| *Enterococcus faecalis* | ATCC 29212 | | 0.5 |
| *Enterococcus faecalis* | ATCC 51299 | VRE | 1 |
| *Streptococcus* | ATCC 49619 | | 0.5 |
| *Enterococcus hirae* | ATCC 8043 | | 0.5 |
| *Bacillus subtilis* | ATCC 6633 | | 0.03 |
| *Escherichia coli* | ATCC 25922 | | >64 |

Taken all together, excellent efficacy of primycin was observed against all the bacteria tested, and this efficacy was not affected by any resistances to any tested antibiotics or by the mechanisms of said resitances.

Based on the facts above it can be established that primycin can be applied excellently against methicillin-resistant *Staphylococcus aureus* (MRSA), methicillin-resistant coagulase--negative *Staphylococcus sp.* (MRCoNS), vancomycin-intermediate of vancomycin-resistant *Staphylococcus aureus* (VISA, VRSA), mupirocin-resistant *Staphylococcus sp.,* vancomycin--resistant *Enterococcus sp.* (VRE) and penicillin-resistant *Streptococcus pneumoniae* strains.

### Examples:

### Example 1

### Ointment containing primycin as active substance applicable on the surfaces of skin or mucous membrane

Composition:

| | |
|---|---|
| Primycin | 1.0 mg/g |
| White vaseline | 700 mg/g |
| Wool fat | 50 mg/g |
| Liquid paraffin | 300 mg/g |

Preparation:
   Primycin is suspended in liquid paraffin at 60-65°C. White vaseline and wool fat are separately warmed to 60-65°C. The two solutions are homogenized and cooled to 35-40°C with continuous stirring.

### Example 2

### Other ointment compositions applicable on the surfaces of skin or mucous membrane

These ointments are prepared according to the procedure described in example 1 applying one of the following active ingredients in the following concentration ranges.

Composition:

| | |
|---|---|
| Primycin or | |
| Primycin component or | |
| Combination of primycin components | 0.01 - 10.0 mg/g |
| White vaseline | 600-800 mg/g |
| Wool fat | 50-60 mg/g |
| Liquid paraffin | 200-400 mg/g |

### Example 3

### Aqueous suspensions containing primycin as active substance, applicable on the surfaces of skin or mucous membrane

Composition:

| | |
|---|---|
| Primycin | 0.5 mg/g |
| Poly(vinyl-alcohol) | 100 mg/g |
| NaH₂PO₄. 0 H₂O | 0.5 mg/g |
| Na₂HPO₄. 2 H₂O | 4.5 mg/g |
| Polysorbate | 6.0 mg/g |
| Disodium edetate | 1.0 mg/g |
| Sodium chloride | 5.0 mg/g |
| Water for injection | ad 1000 g |

Preparation:
1. Sodium dihydrogen phosphate is dissolved in water for injection, with stirring.
2. Disodium hydrogen phosphate is dissolved in the stirred solution.
3. Sodium chloride is dissolved in the stirred solution.
4. Disodium edetate is dissolved in the stirred solution.
5. Polysorbate is mixed with continuous stirring and the solution is adjusted with water for injection.
6. The active ingredient is suspended in the mixture with continuous, intensive stirring.

### Example 4

### Other aqueous suspensions applicable on the surfaces of skin or mucous membrane

These suspensions are prepared according to the procedure described in example 3 applying one of the following active ingredients in the following concentration ranges.

Composition:

| | |
|---|---|
| Primycin or | |
| Primycin component or | |
| Combination of primycin components | 0.01 -10.0 mg/g |
| Poly(vinyl-alcohol) | 50-150 mg/g |
| NaH₂PO₄ . 0 H₂O | 0. 2-1.2 mg/g |
| Na₂HPO₄. 2 H₂O | 4.0-5.0 mg/g |
| Polysorbate | 6-7 mg/g |
| Disodium edetate | 1.0-1.5 mg/g |
| Sodium chloride | 5.0-6.0 mg/g |
| Water for injection | ad 1000 g |

### Example 5

### Alcoholic gel containing primycin as active substance

Composition:

| | |
|---|---|
| Primycin | 0.5 mg/g |
| Carbomera | 10.0 mg/g |
| Triethanolamine | 15.0 mg/g |
| Polysorbate | 15.0 mg/g |
| Isoadipate | 60 mg/g |
| Ethanol (96%) | 500 mg/g |
| Purified water | 300 mg/g |

Preparation:
   Carbomera is swollen in isoadipate. Primycin is dissolved in the mixture of ethanol and purified water with moderate heating. The solution is stirred till it cools down to room temperature. To this solution Carbomera isoadipate dispersion is added. Then triethanolamine is added and the resultant dispersion is stirred till full gelation.

### Example 6

### Other alcoholic gel compositions

These alcoholic gels are prepared according to the procedure described in example 5 applying one of the following active ingredients in the following concentration ranges.

Ingredients:

| | |
|---|---|
| Primycin or | |
| Primycin component or | |
| Combination of primycin components | 0.01 - 10.0 mg/g |
| Carbomera | 7.0-14 mg/g |
| Triethanolamine | 15-20 mg/g |
| Polysorbate | 15-20 mg/g |
| Isoadipate | 50-70 mg/g |
| Ethanol (96%) | 400-600 mg/g |
| Purified water | 300-400 mg/g |

### Advantages of the invention

Spreading of methicillin- and /or vancomycin-resistant Gram-positive pathogens substantially accelerated in the last decades, which rendered all antibiotics affecting these bacteria very valuable both socially and economically.

## Claims

1. Primycin or a primycin component represented by the following general formula (I) 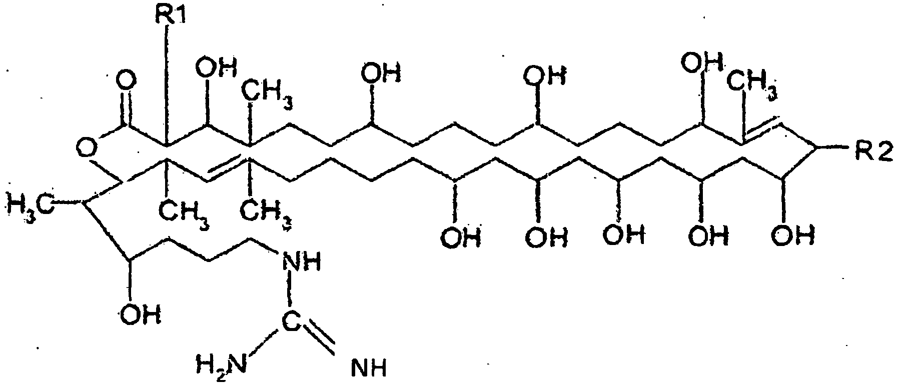 wherein R1 is n-butyl, n-pentyl or n-hexyl and R2 is arabinosyl, H or OH,
which primycin component is in basic form or in salt form,
or a combination of said primycin components for use in the treatment or prevention of infections caused by methicillin-resistant *Staphylococcus aureus* (MRSA), methicillin-resistant coagulase-negative *Staphylococcus sp.* (MRCoNS), vancomycin-intermediate or vancomycin--resistant *Staphylococcus aureus* (VISA, VRSA), mupirocin-resistant *Staphylococcus sp.,* vancomycin-resistant *Enterococcus sp.* (VRE) or penicillin-resistant *Streptococcus pneumoniae* strains.

2. Primycin or a primycin component represented by general formula (I) or a combination of primycin components for use according to claim 1 wherein the primycin components are in the form of sulphate or acetate salt.

3. Primycin or a primycin component represented by general formula (I) or a combination of primycin components for use according to claim 1 or 2 where the combination of primycin components comprises 2, 3, 4, 5, 6, 7, 8 or 9 components selected from the following group:
chinopricin (component A1, where R1 = n-butyl; R2 = arabinosyl)
midopricin (component A2, where R1 = n-pentyl; R2 = arabinosyl)
metipricin (component A3, where R1 = n-hexyl; R2 = arabinosyl)
hydropricin (component B1, where R1 = n-butyl; R2 = H)
hymipricin (component B2, where R1 = n-pentyl; R2 = H)
hymetipricin (component B3, where R1 = n-hexyl; R2 = H)
oxypricin (component C1, where R1 = n-butyl; R2 = OH)
oxymipricin (component C2, where R1 = n-pentyl; R2 = OH)
oxymetipricin (component C3, where R1 = n-hexyl; R2 = OH).

4. Primycin or a primycin component represented by general formula (I) or a combination of primycin components for use according to claim 1 or 3 where the combination of primycin components is selected from the following group: A1 and B1 in a ratio of 1:3 to 3:1, A1 and C1 in a ratio of 1:3 to 3:1, B1 and C1 in a ratio of 1:3 to 3:1 or A1, B1 and C1 in a ratio of 4:3:3 to 7:2:1.

5. Primycin or a primycin component represented by general formula (I) or a combination of primycin components for use according to claim 4 where the combination of primycin components is selected from the following group: A1 and B1 in a ratio of 1:3, 1:1 or 3:1, A1 and C1 in a ratio of 1:3, 1:1 or 3:1, B1 and C1 in a ratio of 1:3, 1:1 or 3:1 or A1, B1 and C1 in a ratio of 4:3:3,6:2:2 or 7:2:1.

6. Pharmaceutical composition containing as active substance primycin or a primycin component represented by general formula (I) or a combination of primycin components for use according to any of claims 1 to 5, together with at least one carrier or vehicle commonly used in the pharmaceutical industry.

7. The pharmaceutical composition for use according to claim 6 which contains primycin or a primycin component represented by general formula (I) or a combination of primycin components in a concentration range of 0.01 mg/g to 10mg/g.

8. The pharmaceutical composition for use according to claim 7 which contains primycin or a primycin component represented by general formula (I) or a combination of primycin components in a concentration range of 0.1 mg/g to 1.0 mg/g.

9. The pharmaceutical composition for use according to any of claims 6 to 8 which is applicable on the surface of skin or mucous membrane of a patient or hospital personnel, which pharmaceutical composition is preferably in the form of cream, ointment, gel, solution, suspension, lotion, nasal drops, nasal spray, ear drops or eye drops.

10. The antibacterial composition containing primycin or a primycin component represented by general formula (I) or a combination of primycin components for use according to any of claims 1 to 5, together with at least one carrier or vehicle commonly used in sanitary and hygienic products.

## Patentansprüche

1. Primycin oder eine Primycin-Komponente dargestellt durch die allgemeine Formel (I) 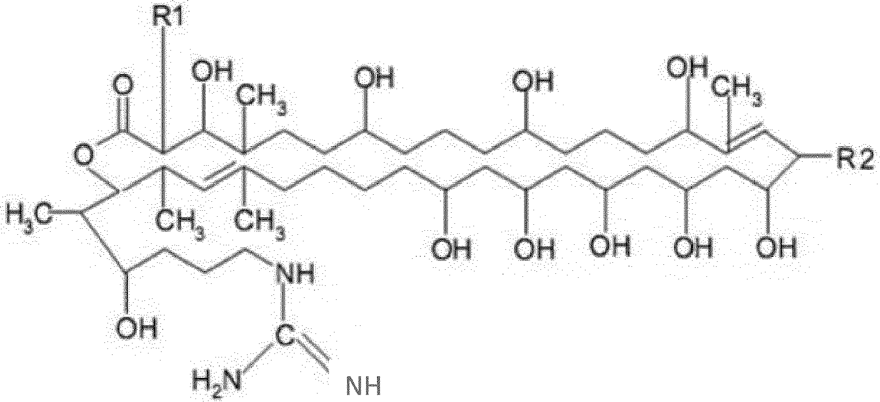 wobei R1 für n-Butyl, n-Pentyl oder n-Hexyl und R2 für Arabinosyl, H oder OH stehen,
welche Primycin-Komponente in Form einer Basis oder in Form eines Salzes steht,
oder eine Kombination von diesen Primycin-Komponenten zur Verwendung in der Behandlung oder Prävention von Infektionen, welche durch Methicillin-resistente *Staphylococcus aureus* (MRSA), Methicillin-resistente Koagulase-negative *Staphylococcus sp.* (MRCoNS), Vancomycin-intermediäre oder Vancomycin-resistente *Staphylococcus aureus* (VISA, VRSA), Mupirocin-resistente *Staphylococcus sp.,* Vancomycin-resistente *Enterococcus sp.* (VRE) oder Penicillin-resistente *Streptococcus pneumoniae* Stämme ausgelöst wurden.

2. Primycin oder eine Primycin-Komponente dargestellt durch die allgemeine Formel (I) oder eine Kombination von Primycin-Komponenten für die Verwendung nach Anspruch 1 wobei die Primycin-Komponenten in Form von Sulphat- oder Acetatsalzen sind.

3. Primycin oder eine Primycin-Komponente dargestellt durch die allgemeine Formel (I) oder eine Kombination von Primycin-Komponenten für die Verwendung nach Anspruch 1 oder 2, wobei die Kombination von Primycin-Komponenten 2, 3, 4, 5, 6, 7, 8 oder 9 Komponenten enthalten, welche aus der folgenden Gruppe ausgewählt sind:
Chinopricin (Komponente A1, wobei R1 = n-Butyl; R2 = Arabinosyl)
Midopricin (Komponente A2, wobei R1 = n-Pentyl; R2 = Arabinosyl)
Metipricin (Komponente A3, wobei R1 = n-Hexyl; R2 = Arabinosyl)
Hydropricin (Komponente B1, wobei R1 = n-Butyl; R2 = H)
Hymipricin (Komponente B2, wobei R1 = n-Pentyl; R2 = H)
Hymetipricin (Komponente B3, wobei R1 = n-Hexyl; R2 = H)
Oxypricin (Komponente C1, wobei R1 = n-Butyl; R2 = OH)
Oxymipricin (Komponente C2, wobei R1 = n-Pentyl; R2 = OH)
Oxymetipricin (Komponente C3, wobei R1 = n-Hexyl; R2 = OH)

4. Primycin oder eine Primycin-Komponente dargestellt durch die allgemeine Formel (I) oder eine Kombination von Primycin-Komponenten für die Verwendung nach Anspruch 1 oder 3, wobei die Kombination von Primycin-Komponenten aus der folgenden Gruppe ausgewählt ist: A1 und B1 im Verhältnis von 1:3 bis 3:1, A1 und C1 im Verhältnis von 1:3 bis 3:1, B1 und C1 im Verhältnis von 1:3 bis 3:1 oder A1, B1 und C1 im Verhältnis von 4:3:3 bis 7:2:1.

5. Primycin oder eine Primycin-Komponente dargestellt durch die allgemeine Formel (I) oder eine Kombination von Primycin-Komponenten für die Verwendung nach Anspruch 4, wobei die Kombination von Primycin-Komponenten aus der folgenden Gruppe ausgewählt ist: A1 und B1 im Verhältnis von 1:3, 1:1 oder 3:1, A1 und C1 im Verhältnis von 1:3, 1:1 oder 3:1, B 1 und C1 im Verhältnis von 1:3, 1:1 oder 3:1 oder A1, B 1 und C1 im Verhältnis 4:3:3, 6:2:2 oder 7:2:1.

6. Pharmazeutische Zusammensetzung enthaltend als Wirkstoff Primycin oder eine Primycin-Komponente dargestellt durch die allgemeine Formel (I) oder eine Kombination von Primycin-Komponenten für die Verwendung nach einem der Ansprüchen 1 bis 5, zusammen mit wenigstens einem in der pharmazeutischen Industrie üblichen Trägerstoff oder Bindemittel.

7. Die pharmazeutische Zusammensetzung für die Verwendung nach Anspruch 6 enthaltend Primycin oder eine Primycin-Komponente dargestellt durch die allgemeine Formel (I) oder eine Kombination von Primycin-Komponenten in einem Konzentrationsbereich von 0,01 mg/g bis 10 mg/g.

8. Die pharmazeutische Zusammensetzung für die Verwendung nach Anspruch 7 enthaltend Primycin oder eine Primycin-Komponente dargestellt durch die allgemeine Formel (I) oder eine Kombination von Primycin-Komponenten in einem Konzentrationsbereich von 0,1 mg/g bis 1,0 mg/g.

9. Die pharmazeutische Zusammensetzung für die Verwendung nach einem der Ansprüche 6 bis 8, welche an der Oberfläche der Haut oder Mukosamembran eines Patienten oder Krankenhausmitarbeiters verwendbar ist, wobei die pharmazeutische Zusammensetzung bevorzugt in Form von Krem, Salbe, Gel, Lösung, Suspension, Lotion, Nasentropfen, Nasenspray, Ohrentropfen oder Augentropfen vorliegt.

10. Antibakterielle Zusammensetzung enthaltend Primycin oder eine Primycin-Komponente dargestellt durch die allgemeine Formel (I) oder eine Kombination von Primycin-Komponenten für die Verwendung nach einem der Ansprüche 1 bis 5, zusammen mit wenigstens einem in sanitären und hygienischen Produkten üblichen Trägerstoff oder Bindemittel.

## Revendications

1. Primycine ou un composant de primycine représenté par la formule générale (I) suivante 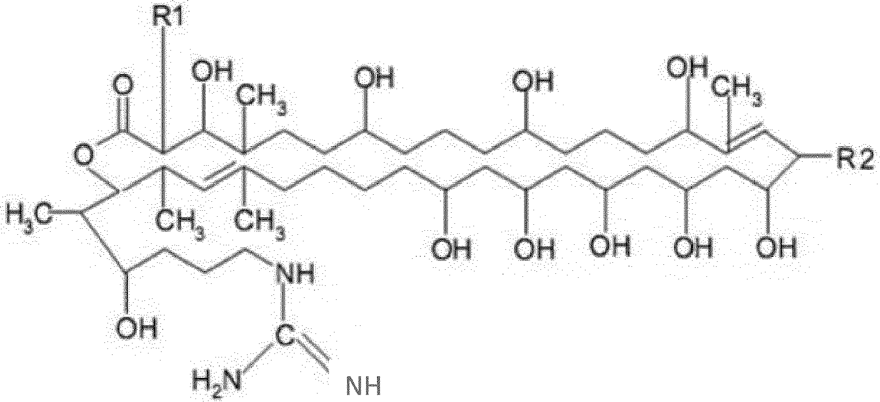 dans laquelle R1 est n-butyle, n-pentyle ou n-héxyle et R2 est arabinosyle, H ou OH,
lequel composant de primycine est sous forme de base ou sous forme de sel,
ou une combinaison desdits composants de primycine pour l'utilisation dans le traitement ou la prévention d'infections causés par des souches de *Staphylococcus aureus* résistants à la méticilline (SARM), *Staphylococcus sp.* à coagulase négative résistants à la méticilline (SCoNRM), *Staphylococcus aureus* intermédiaires à la vancomycine ou résistants à la vancomycine (SAIV, SARV), *Staphylococcus sp.* résistants à la mupirocine, *Enterococcus sp.* résistants à la vancomycine (ERV) ou *Streptococcus pneumoniae* résistants à la penicilline.

2. Primycine ou un composant de primycine représenté par la formule générale (I) ou une combinaison des composants de primycine pour l'utilisation selon la revendication 1 où les composants de primycine sont sous la forme de sel de sulfate ou de sel d'acétate.

3. Primycine ou un composant de primycine représenté par la formule générale (I) ou une combinaison des composants de primycine pour l'utilisation selon la revendication 1 ou 2 où la combinaison des composants de primycine comprend 2, 3, 4, 5, 6, 7, 8 ou 9 composants sélectionnés parmi le groupe suivant:
chinopricine (composant A1, où R1 = n-butyle; R2 = arabinosyle)
midopricine (composant A2, où R1 = n-pentyle; R2 = arabinosyle)
metipricine (composant A3, où R1 = n-héxyle; R2 = arabinosyle)
hydropricine (composant B1, où R1 = n-butyle; R2 = H)
hymipricine (composant B2, où R1 = n-pentyle; R2 = H)
hymetipricine (composant B3, où R1 = n-héxyle; R2 = H)
oxypricine (composant C1, où R1 = n-butyle; R2 = OH)
oxymipricine (composant C2, où R1 = n-pentyle; R2 = OH)
oxymetipricine (composant C3, où R1 = n-héxyle; R2 = OH)

4. Primycine ou un composant de primycine représenté par la formule générale (I) ou une combinaison des composants de primycine pour l'utilisation selon la revendication 1 ou 3 où la combinaison des composants de primycine est sélectionnée parmi le groupe suivante: A1 et B1 dans une proportion de 1:3 à 3:1, A1 et C1 dans une proportion de 1:3 à 3:1, B1 et C1 dans une proportion de 1:3 à 3:1 ou A1, B1 et C1 dans une proportion de 4:3:3 à 7:2:1.

5. Primycine ou un composant de primycine représenté par la formule générale (I) ou une combinaison des composants de primycine pour l'utilisation selon la revendication 4 où la combinaison des composants de primycine est sélectionnée parmi le groupe suivant: A1 et B1 dans une proportion de 1:3, 1:1 ou 3:1, A1 et C1 dans une proportion de 1:3, 1:1 ou 3:1, B1 et C1 dans une proportion de 1:3, 1:1 ou 3:1 ou A1, B1 et C1 dans une proportion de 4:3:3, 6:2:2 ou 7:2:1.

6. Composition pharmaceutique contenant, comme substance active, de la primycine ou un composant de primycine représenté par la formule générale (I) ou une combinaison des composants de primycine pour l'utilisation selon l'une quelconque des revendications 1 à 5, en association avec au moins un véhicule ou excipient classiquement utilisé dans l'industrie pharmaceutique.

7. La composition pharmaceutique pour l'utilisation selon la revendication 6 contenant de la primycine ou un composant de primycine représenté par la formule générale (I) ou une combinaison des composants de primycine dans une gamme de concentration de 0.01 mg/g à 10 mg/g.

8. La composition pharmaceutique pour l'utilisation selon la revendication 7 contenant de la primycine ou un composant de primycine représenté par la formule générale (I) ou une combinaison des composants de primycine dans une gamme de concentration de 0.1 mg/g à 1.0 mg/g.

9. La composition pharmaceutique pour l'utilisation selon l'une quelconque des revendications 6 à 8, qui peut être appliquée sur la surface de la peau ou de la membrane muqueuse d'un patient ou d'un personnel hospitalier, laquelle composition pharmaceutique est préférablement sous la forme de crème, onguent, gel, solution, suspension, lotion, goutte nasale, spray nasal, goutte d'oreille ou goutte d'oeil.

10. La composition antibactérienne contenant de la primycine ou un composant de primycine représenté par la formule générale (I) ou une combinaison des composants de primycine pour l'utilisation selon l'une quelconque des revendications 1 à 5, en association avec au moins un véhicule ou excipient classiquement utilisé dans les produits sanitaires et hygiéniques.
